# EUROPEAN PATENT APPLICATION

(11) **EP 4 799 720 A1**
(43) Date of publication of application: **02.09.2026**
(21) Application number: 24881992.2
(22) Date of filing: 13.08.2024
(51) Int. Cl.: B01J 8/04, B01J 8/00, B01J 19/00, C07C 1/12, C07C 9/04

(54) **GENERATION DEVICE**

(30) Priority: 24.10.2023 JP 2023182608
(71) Applicant: Kanadevia Corporation, Osaka-shi, Osaka 559-8559 (JP)
(72) Inventor: ECHIGO, Takumi, Osaka-shi, Osaka 559-8559 (JP)
(74) Representative: Grünecker Patent- und Rechtsanwälte PartG mbB
(86) International application number: PCT/JP2024/028908
(87) International publication number: WO 2025/088875

(57) **Abstract**

Provided is a technique to generate high concentration product gas. A generation device includes: a plurality of reactors that include a first reactor and a second reactor to generate product gas by catalytic reaction of source gas; a gas passage in which the source gas flows through the plurality of reactors; a circulation passage in which heating medium for adjusting temperature inside the plurality of reactors circulates through the plurality of reactors; and a temperature adjusting unit which is disposed in the circulation passage and adjusts temperature of the heating medium. In a direction in which the source gas flows in the gas passage, the second reactor is disposed behind the first reactor, and in a direction in which the heating medium circulates in the circulation passage, the second reactor is disposed behind the temperature adjusting unit, and the first reactor is disposed behind the second reactor.

## Description

### TECHNICAL FIELD

The present invention relates to a generation device.

### BACKGROUND ART

Patent Literature 1, for example, discloses a technique related to a generation device and a generation method for generating a product gas using the exothermic reaction of a reactant in a gas state. The generation device according to Patent Literature 1 includes a first step reaction tower where hydrogen and carbon dioxide are supplied causing an exothermic reaction, and a second step reaction tower, where an exothermic reaction is caused using the unreacted hydrogen and the carbon dioxide to generate the product gas. In the generation device according to Patent Literature 1, heating medium oil, heated by a heating medium oil heater, is sent to the first step reaction tower, and the heating medium oil that passed through the first step reaction tower is sent to the second step reaction tower.

### CITATION LIST

### PATENT LITERATURE

Patent Literature 1: Japanese Patent No. 6984098

### SUMMARY OF INVENTION

### TECHNICAL PROBLEM

The generation of methane gas (product gas) is caused by an exothermic reaction, so if the temperature of the heating medium oil is lowered, reaction progresses such that a concentration of the product gas increases due to chemical equilibrium. However the heating medium oil that is sent to the second reaction tower has been heated to a high temperature by the exothermic reaction in the first step reaction tower. Therefore it is difficult for the concentration of the product gas in the second step reaction tower to become high, due to chemical equilibrium.

It is an object of the present invention to provide a technique to generate a high concentration product gas.

### SOLUTION TO PROBLEM

To solve the above problem, the present invention is a generation device including: a plurality of reactors that includes a first reactor and a second reactor to generate product gas by catalytic reaction of source gas; a gas passage in which the source gas flows through the plurality of reactors; a circulation passage in which heating medium for adjusting temperature inside the plurality of reactors circulates through the plurality of reactors; and a temperature adjusting unit which is disposed in the circulation passage and adjusts temperature of the heating medium. In a direction in which the source gas flows in the gas passage, the second reactor is disposed behind the first reactor, and in a direction in which the heating medium circulates in the circulation passage, the second reactor is disposed behind the temperature adjusting unit, and the first reactor is disposed behind the second reactor.

The heating medium of which temperature has been adjusted by the temperature adjusting unit passes through the second reactor, then passes through the first reactor. This prevents the temperature of the heating medium passing through the second reactor from being excessively high, and makes it easy to appropriately maintain the temperature inside the second reactor. By appropriately maintaining the temperature inside the second reactor (e.g. at low temperature), the concentration of product gas generated in the second reactor increases due to chemical equilibrium, and the generation device can generate a high concentration product gas.

The capacity of the first reactor and the capacity of the second reactor may be different. The capacity of the second reactor may be smaller than the capacity of the first reactor. If the capacity of the second reactor decreases, the surface area of the second reactor decreases. And if the surface area of the second reactor decreases, heat release of the second reactor can be suppressed, which makes it easier to maintain the heat inside the second reactor at an appropriate temperature. Further, if heat release of the second reactor is suppressed, the heating medium is heated more by the exothermic reaction in the second reactor, whereby high temperature heating medium can be sent to the first reactor. By decreasing the surface area of the second reactor, the cost for materials of the second reactor, the insulating material, and the like, can be reduced.

The capacity of the second reactor may be larger than the capacity of the first reactor. If the capacity of the second reactor is larger than the capacity of the first reactor, the amount of the product gas generated by the second reactor increases, and the second reactor can generate a larger amount of high concentration product gas.

The circulation passage may include: a first passage in which the heating medium, of which temperature has been adjusted by the temperature adjusting unit, flows through the second reactor; and a second passage, which branches from the first passage and in which the heating medium, of which temperature has been adjusted by the temperature adjusting unit, flows bypassing the second reactor. The second passage may merge with the first passage on the downstream side of the second reactor. In the case where the capacity of the second reactor is smaller than the capacity of the first reactor, a pressure loss is generated if the flow rate of the heating medium that passes through the second reactor is too high, hence it is preferable that the flow rate of the heating medium that passes through the second reactor is lower than the flow rate of the heating medium that passes through the first reactor. The circulation passage includes the first passage in which the heating medium passes through the second reactor, and the second passage in which the heating medium flows bypassing the second reactor. Hence the flow rate of the heating medium that passes through the second reactor decreases, and pressure loss is suppressed. The generation device may include a flow rate control unit that controls a flow rate of the heating medium which flows in the first passage, and a flow rate of the heating medium which flows in the second passage. Thereby the flow rate of the heating medium that passes through the second reactor can be controlled.

The generation device may include a second temperature adjusting unit which is disposed in the circulation passage, on the upstream side of the first reactor and on the downstream side of a merged portion of the first passage and the second passage, and which adjusts the temperature of the heating medium. Since the heating medium, of which temperature has been adjusted by the second temperature adjusting unit, is sent to the first reactor, it becomes easier to appropriately maintain the temperature inside the first reactor.

The temperature adjusting unit may adjust the temperature of the heating medium such that the temperature of the heating medium, which flows in the first passage on the upstream side of the second reactor, falls within a first temperature range. The second temperature adjusting unit may adjust the temperature of the heating medium such that the temperature of the heating medium, which flows on the upstream side of the first reactor and on the downstream side of the merged portion, falls within a second temperature range, which is higher than the first temperature range. This makes it easier to maintain the temperature inside the first reactor to be higher than the temperature inside the second reactor.

The circulation passage may include a first passage in which the heating medium flows through the first reactor, and a second passage which branches from the first passage, and in which the heating medium flows bypassing the first reactor. The second passage may merge with the first passage on the downstream side of the first reactor. In the case where the capacity of the first reactor is smaller than the capacity of the second reactor, a pressure loss is generated if the flow rate of the heating medium passing through the first reactor is too high, hence it is preferable that the flow rate of the heating medium that passes through the first reactor is lower than the flow rate of the heating medium that passes through the second reactor. The circulation passage includes the first passage in which the heating medium passes through the first reactor, and the second passage in which the heating medium flows bypassing the first reactor. Hence the flow rate of the heating medium that passes through the first reactor decreases, and pressure loss is suppressed. The generation device may include a flow rate control unit that controls the flow rate of the heating medium which flows in the first passage, and the flow rate of the heating medium which flows in the second passage. Thereby the flow rate of the heating medium that passes through the first reactor can be controlled.

The generation device may include a second temperature adjusting unit which is installed in the circulation passage, and is disposed in the first passage, on the upstream side of the first reactor, and on the downstream side of a branched portion of the first passage and the second passage, and which adjusts the temperature of the heating medium. Since the heating medium, of which temperature has been adjusted by the second temperature adjusting unit, is sent to the first reactor, it becomes easier to appropriately maintain the temperature inside the first reactor.

The temperature adjusting unit may adjust the temperature of the heating medium such that the temperature of the heating medium, which flows on the upstream side of the second reactor, falls within a first temperature range. The second temperature adjusting unit may adjust the temperature of the heating medium such that the temperature of the heating medium, which flows in the first passage on the upstream side of the first reactor, and on the downstream side of the branched portion falls within a second temperature range, which is higher than the first temperature range. This makes it easier to maintain the temperature inside the first reactor to be higher than the temperature inside the second reactor.

At least a part of the heating medium, that flows in the circulation passage on the downstream side of the first reactor and on the upstream side of the second reactor, may be returned to the downstream side of the temperature adjusting unit in the circulation passage, in a state of being cooled by at least one equipment unit. Since the heating medium which passed through the first reactor is at high temperature, the heat of the heating medium, which passed through the first reactor, can be used by at least one equipment unit.

The temperature adjusting unit may include a cooler to cool the heating medium. A part of the heating medium cooled by the cooler may be returned to the circulation passage on the upstream side of the first reactor, and on the downstream side of the second reactor, in a state of being heated by at least one equipment unit. Since the heating medium that passed through the cooler has been cooled, the heating medium which passed through the cooler can be used by at least one equipment unit. If the heated heating medium is returned to the circulation passage on the upstream side of the first reactor and on the downstream side of the second reactor, the heated heating medium can be sent to the first reactor.

The source gas may flow inside the plurality of reactors from gas inlets located on a first side of the plurality of reactors to gas outlets located on a second side of the plurality of reactors. The heating medium may flow inside the plurality of reactors from heating medium inlets located on the second side of the plurality of reactors to heating medium outlets located on the first side of the plurality of reactors. The source gas and the heating medium flow in opposite directions from each other in the multiple reactors.

### ADVANTAGEOUS EFFECTS OF INVENTION

A high concentration product gas can be generated.

### BRIEF DESCRIPTION OF THE DRAWINGS

[Fig. 1] Fig. 1 is a block diagram of a generation device according to Embodiment 1.
[Fig. 2] Fig. 2 is a block diagram of a generation device according to Embodiment 2.
[Fig. 3] Fig. 3 is a block diagram of a generation device according to Embodiment 3.
[Fig. 4] Fig. 4 is a block diagram of a generation device according to Embodiment 4.
[Fig. 5] Fig. 5 is a block diagram of a generation device according to Embodiment 5.
[Fig. 6] Fig. 6 is a block diagram of a generation device according to a modification.

### DESCRIPTION OF EMBODIMENTS

Embodiments of the present invention will now be described. The following embodiments are examples of embodiments of the present invention, and are not intended to limit the technical scope of the present invention to these embodiments.

### <Embodiment 1>

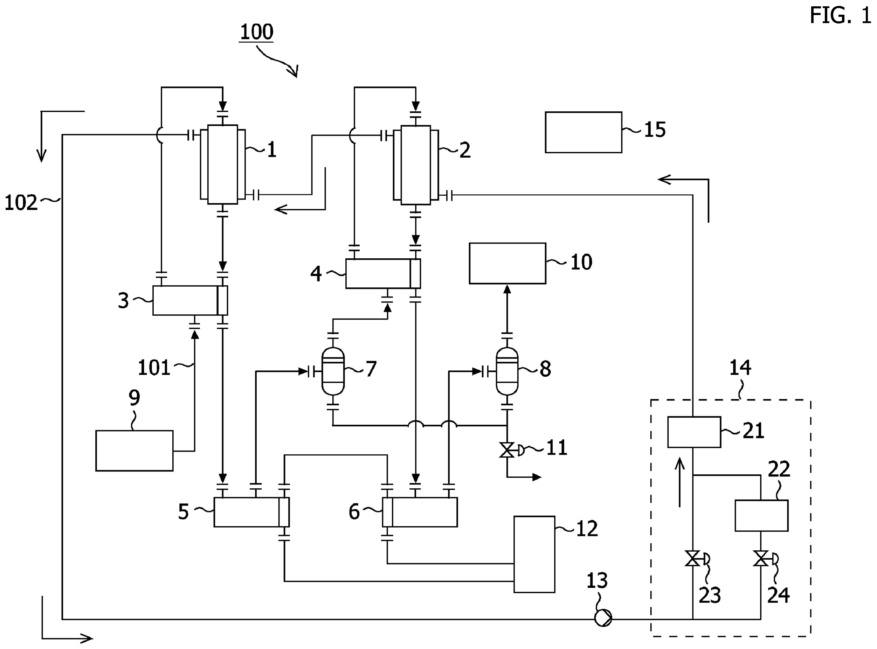

Embodiment 1 will be described with reference to Fig. 1. Fig. 1 is a block diagram of a generation device 100 according to Embodiment 1. The generation device 100 in Fig. 1 generates methane gas, which is a product gas (generation gas), and water, for example, by the exothermic reaction of hydrogen and carbon dioxide in a gas state, which is a source gas (reaction gas). This exothermic reaction is also a reversible reaction. The above exothermic reaction is represented by the following reaction formula.

CO₂+4H₂→CH₄+2H₂O ... (1)

The generation device 100 includes a reactor (first reactor) 1, a reactor (second reactor) 2, economizers 3 and 4, gas cooling heat exchangers 5 and 6, gas-liquid separators 7 and 8, a source gas supply unit 9, and a storage tank 10. The generation device 100 also includes a gas passage 101 in which the source gas flows through the reactors 1 and 2. The reactors 1 and 2, the economizers 3 and 4, the gas cooling heat exchangers 5 and 6, and the gas-liquid separators 7 and 8 are disposed in the gas passage 101. Pipes and valves are also disposed in the gas passage 101.

The source gas is supplied from the source gas supply unit 9 into the reactor 1. The reactor 1 generates product gas by the catalytic reaction of the source gas. The source gas contains hydrogen (H₂) and carbon dioxide (CO₂), for example. The reactor 1 and the economizer 3 are connected, and the economizer 3 and the source gas supply unit 9 are connected. In the economizer 3, heat exchange occurs between the source gas delivered from the source gas supply unit 9 and gas delivered from the reactor 1. The gas delivered from the reactor 1 is a product gas, an unreacted source gas, or a mixed gas of the product gas and the unreacted source gas.

The economizer 3 and the gas cooling heat exchanger 5 are connected. The reactor 1 generates water (vapor) by the catalytic reaction of the source gas. The gas cooling heat exchanger 5 condenses the water (vapor) generated by the reactor 1. The gas cooling heat exchanger 5 and the gas-liquid separator 7 are connected. The gas-liquid separator 7 separates the generated water (liquid) from the product gas and the unreacted source gas.

The reactor 2 and the economizer 4 are connected, and the economizer 4 and the gas-liquid separator 7 are connected. The product gas generated by the reactor 1 and unreacted source gas are delivered to the reactor 2 via the economizer 3, the gas cooling heat exchanger 5, the gas-liquid separator 7 and the economizer 4. The reactor 2 generates the product gas by the catalytic reaction of the source gas. By the reactor 2 generating the product gas from the unreacted source gas, the generation device 100 can generate a high concentration product gas.

In the economizer 4, heat exchange occurs between the gas delivered from the gas-liquid separator 7 and the gas delivered from the reactor 2. The gas delivered from the reactor 2 is a product gas, an unreacted source gas, or a mixed gas of the product gas and the unreacted source gas.

The economizer 4 and the gas cooling heat exchanger 6 are connected, and the gas cooling heat exchanger 6 and the gas-liquid separator 8 are connected. The reactor 2 generates water (vapor) by the catalytic reaction of the source gas. The gas cooling heat exchanger 6 condenses the water (vapor) generated by the reactor 2. The gas-liquid separator 8 separates the generated water (liquid) from the product gas and the unreacted source gas.

The storage tank 10 stores the product gas. The product gas is delivered from the gas-liquid separator 8 to the storage tank 10. A removal unit may be disposed between the gas-liquid separator 8 and the storage tank 10. The product gas may be delivered to the storage tank 10 after the removal unit removes the unreacted source gas. Drain valves 11 are disposed on the gas-liquid separators 7 and 8 for discharging the generated water. The drain valve 11 may be a type that opens/closes using the buoyancy of a floating tool, (e.g. drain trap), or may be a type that electrically detects the water level and opens/closes a solenoid valve.

The generation device 100 includes a chiller 12. The chiller 12 cools cooling water (coolant) to condense the generated water in the gas cooling heat exchangers 5 and 6. The gas cooling heat exchangers 5 and 6 and the chiller 12 are connected to each other via pipes in which the cooling water flows. The cooling water, cooled by the chiller 12, returns to the chiller 12 via the heat exchangers for the cooling gas 5 and 6.

The reactors 1 and 2 are filled with catalyst in advance. The catalyst may be any catalyst that accelerates the reaction formula (1). For example, the catalyst includes stabilized zirconia carrier and Ni carried by the stabilized zirconia carrier. The stabilized zirconia carrier includes a tetragonal system and/or a cubic system crystal structure in which the stabilizing element dissolves. For the stabilizing element, at least one type of transition element is selected from a group of Mn, Fe and Co.

The generation device 100 includes a pump 13, a temperature adjusting unit 14, and a control unit 15. The generation device 100 also includes a circulation passage 102 in which a heating medium for adjusting the temperature inside the reactors 1 and 2 circulates through the reactors 1 and 2. The heating medium used here is heating medium oil, for example, but is not limited to this, and may be water (vapor). The reactors 1 and 2, the pump 13 and the temperature adjusting unit 14 are disposed in the circulation passage 102. Pipes and valves are also disposed on the circulation passage 102.

The control unit 15 is a controller to control the operation of the generation device 100 in general. The control unit 15 may be a dedicated apparatus, or a general purpose computer. The control unit 15 includes such hardware resources as a processor (CPU), memory, storage, and communication I/F. The memory may be RAM. The storage may be a non-volatile storage device (e.g. ROM, flash memory). The functions of the control unit 15 are implemented by developing programs stored in the storage in memory, and the processor executing programs. The configuration of the control unit 15 is not limited to this. For example, all or a part of the functions may be implemented by such circuits as ASIC and FPGA, or all or a part of the functions may be executed by a cloud server or other devices. The control unit 15 also controls the source gas supply unit 9. By the control unit 15 controlling the operation of the source gas supply unit 9, the source gas supply unit 9 starts supplying the source gas to the reactor 1. The source gas supply unit 9 may operate independently from the control unit 15.

The reactors 1 and 2 are shell-and-tube type heat exchangers, for example. The heating medium for causing heat exchange with a heating portion inside the reactor 1 can flow into or flow out of the shell of the reactor 1. The heating medium for causing heat exchange with the heating portion inside the reactor 2 can flow into or flow out of the shell of the reactor 2. By the heating medium flowing into the shells of the reactors 1 and 2, the heating medium can be circulated inside the reactors 1 and 2, whereby the temperature inside the reactors 1 and 2 is adjusted. The shell of the reactor 1 and the shell of the reactor 2 are connected via a pipe in which the heating medium flows. The reactors 1 and 2 are not limited to the shell-and-tube type heat exchangers, but may be plate type heat exchangers, or heat exchangers of other types.

The source gas flows inside the reactors 1 and 2, from gas inlets located on a first side of the reactors 1 and 2 toward gas outlets located on a second side of the reactors 1 and 2. The heating medium flows inside the reactors 1 and 2, from heating medium inlets located on a second side of the reactors 1 and 2 toward heating medium outlets located on the first side of the reactors 1 and 2. This means that the source gas and the heating medium flow inside the reactors 1 and 2 in opposite directions from each other.

The pump 13 drives the heating medium to flow in the circulation passage 102. The temperature adjusting unit 14 adjusts the temperature of the heating medium that flows in the circulation passage 102. In the circulation passage 102, the temperature adjusting unit 14 is disposed on the downstream side of the reactor 1 and on the upstream side of the reactor 2. The temperature adjusting unit 14 includes a heating unit (heater) 21, a cooling unit (cooler) 22, and regulating valves (control valves) 23 and 24. The shell of the reactor 1 is connected to the heater 21 and the cooler 22 via a pipe in which the heating medium flows. The shell of the reactor 2 and the heater 21 are connected via a pipe in which the heating medium flows. The cooler 22 cools the heating medium which passed through the reactors 1 and 2. In the pipe connecting the shell of the reactor 1 to the heater 21 and the cooler 22, a pump 13 is disposed to circulate the heating medium in the circulation passage 102. The regulating valves 23 and 24 are disposed in the pipe in which the heating medium flows. By opening/closing the regulating valves 23 and 24, the heating medium which passed through the reactor 1 can be delivered to the reactor 2 via the cooler 22, or delivered to the reactor 2 bypassing the cooler 22.

In the case where the regulating valve 23 is opened and the regulating valve 24 is closed, the heating medium is delivered to the reactor 2 via the heater 21. When the heater 21 is driven, the heating medium is heated by the heater 21, and the heated heating medium is delivered to the reactor 2. The control unit 15 may control the driving/stopping of the heater 21. In the case where the regulating valve 23 is closed and the regulating valve 24 is opened, the heating medium is delivered to the reactor 2 via the heater 21 and the cooler 22. In the state where the heater 21 is stopped, the heating medium is cooled by the cooler 22, and the cooled heating medium is delivered to the reactor 2. When the heating medium, of which temperature has been adjusted by the temperature adjusting unit 14, passes through the reactor 2, the temperature inside the reactor 2 is adjusted by the heating medium. The temperature adjusting unit 14 is an example of the first temperature adjusting unit. Supply of the source gas to the reactor 1 may be started after the driving of the pump 13 and the heater 21 is started. Or the driving of the pump 13 and the heater 21 may be started after the supply of the source gas to the reactor 1 is started.

In the direction in which the source gas flows in the gas passage 101, the reactor 2 is disposed behind the reactor 1. This means that in the direction in which the source gas flows in the gas passage 101, the reactor 1 is disposed first, and the reactor 2 is disposed last. Further, in the direction in which the heating medium circulates to the circulation passage 102, the reactor 2 is disposed behind the temperature adjusting unit 14, and the reactor 1 is disposed behind the reactor 2. The heating medium, of which temperature has been adjusted by the temperature adjusting unit 14, passes through the reactor 2 first, and then passes through the reactor 1. Since the heating medium, which has been heated by the exothermic reaction in the reactor 2, passes through the reactor 1, the temperature inside the reactor 1 is adjusted by the heating medium.

If the heating medium, of which temperature has been adjusted, passes through the reactor 1 first and then passes through the reactor 2, for example, the temperature of the heating medium that passes through the reactor 2 becomes high because of the exothermic reaction inside the reactor 1, which makes the temperature inside the reactor 2 excessively high. If the temperature inside the reactor 2 becomes excessively high, the concentration of the product gas decreases due to chemical equilibrium. In the case of the generation device 100 according to Embodiment 1, the heating medium, of which temperature has been adjusted, passes through the reactor 2 first and then passes through the reactor 1, therefore the temperature of the heating medium which passes through the reactor 2 does not become excessively high, which makes it easier to appropriately maintain the temperature inside the reactor 2. By appropriately maintaining the temperature inside the reactor 2 (e.g. low temperature), the concentration of the product gas generated in the reactor 2 increases due to chemical equilibrium, and the generation device 100 can generate high concentration product gas.

### <Embodiment 2>

Embodiment 2 will be described. In Embodiment 2, a composing element the same as Embodiment 1 is denoted with a same reference sign, and description thereof will be omitted. The generation devices 100 according to Embodiments 1 and 2 may be combine as needed.

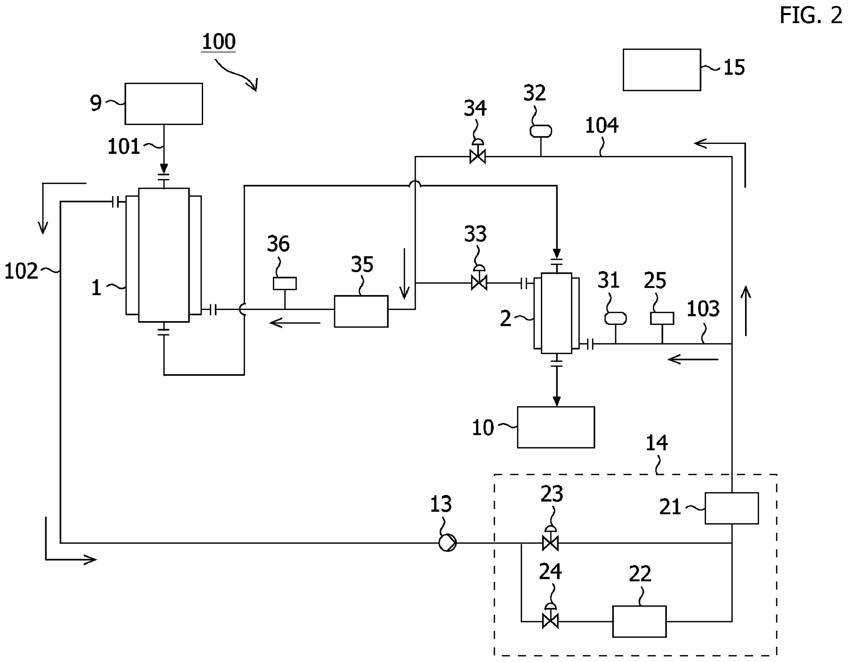

Fig. 2 is a block diagram of the generation device 100 according to Embodiment 2. The generation device 100 includes reactors 1 and 2, economizers 3 and 4, gas cooling heat exchangers 5 and 6, gas-liquid separators 7 and 8, a source gas supply unit 9, and a storage tank 10. The generation device 100 includes a drain valve 11, a chiller 12, a pump 13, a temperature adjusting unit 14, a control unit 15, a gas passage 101, and a circulation passage 102. In Fig. 2, the economizers 3 and 4, the gas cooling heat exchangers 5 and 6, the gas-liquid separators 7 and 8, the drain valve 11 and the chiller 12 are omitted.

In Embodiment 2, the capacity of the reactor 1 and the capacity of the reactor 2 are different, with the capacity of the reactor 2 smaller than the capacity of the reactor 1. The ratio of the capacity of the reactor 1 and the capacity of the reactor 2 is not especially limited. The ratio of the capacity of the reactor 1 and the capacity of the reactor 2 may be determined by experiment or simulation. The circulation passage 102 includes: a passage (first passage) 103 in which the heating medium, of which temperature has been adjusted by the temperature adjusting unit 14, flows through the reactor 2; and a passage (second passage) 104, which branches from the passage 103 and in which the heating medium, of which temperature has been adjusted by the temperature adjusting unit 14, flows bypassing the reactor 2. The passage 104 merges with the passage 103 on the downstream side of the reactor 2 in the passage 103.

In Embodiment 2, the capacity of the reactor 1 and the capacity of the reactor 2 are set based on the difference between the reaction amount of the reactor 1 and the reaction amount of the reactor 2. Since the balance between the reaction amount of the reactor 1 and the reaction amount of the reactor 2 can be changed by design, the capacity of the reactor 2 can be smaller than the capacity of the reactor 1. For example, if it is designed such that the reactor 1 generates relatively high concentration product gas, and the reactor 2 generates product gas by refining a small amount of unreacted source gas, the reaction amount of the reactor 2 is smaller than the reaction amount of the reactor 1, that is, the capacity of the reactor 2 can be smaller than the capacity of the reactor 1.

If the capacity of the reactor 2 decreases, the surface area of the reactor 2 decreases. And as the surface area of the reactor 2 becomes smaller, the released heat amount of the reactor 2 decreases. By decreasing the surface area of the reactor 2, the heat released from the reactor 2 can be suppressed, which makes it easier to appropriately maintain the temperature inside the reactor 2. Further, by suppressing the heat released from the reactor 2, the heating medium is warmed more so by the exothermic reaction in the reactor 2, hence a high temperature heating medium can be delivered to the reactor 1. If the surface of the reactor 2 is decreased, the cost (apparatus cost) for material of the reactor 2, the insulating material, and the like, can be reduced.

In Embodiment 2, the flow rate of the heating medium which passes through the reactor 1, and the flow rate of the heating medium which passes through the reactor 2 are set based on the difference between the capacity of the reactor 1 and the capacity of the reactor 2. In the case where the capacity of the reactor 2 is smaller than the capacity of the reactor 1, it is preferable that the flow rate of this heating medium which passes through the reactor 2 is lower than the flow rate of the heating medium which passes through the reactor 1. For example, in a case where the flow rate of the heating medium which flows into the shell of the reactor 2 is excessively high, a pressure loss is generated, and load on the pump 13 increases thereby. In Embodiment 2, the circulation passage 102 includes a passage 103 in which the heating medium flows through the reactor 2, and a passage 104 in which the heating medium flows bypassing the reactor 2. Thereby the flow rate of the heating medium which passes through the reactor 2 decreases and a pressure loss is suppressed, and load on the pump 13 is decreased. As a result, power to be supplied to the pump 13 can be reduced.

The generation device 100 includes a thermometer 25, which is disposed in the passage 103. The thermometer 25 measures the temperature of the heating medium that flows in the passage 103. The thermometer 25 may be disposed in the passage 103 on the upstream side of the reactor 2. The thermometer 25 may measure the temperature of the heating medium that flows in the passage 103 on the upstream side of the reactor 2. In the configuration example illustrated in Fig. 2, the thermometer 25 is disposed in the passage 103 on the upstream side of the reactor 2, and on the downstream side of the temperature adjusting unit 14. In this case, the thermometer 25 measures the temperature of the heating medium that flows in the passage 103 on the upstream side of the reactor 2, and on the downstream side of the temperature adjusting unit 14. The data measured by the thermometer 25 (measured values of the temperature of heating medium) is sent to the control unit 15. The control unit 15 acquires the data measured by the thermometer 25.

The generation device 100 includes flow meters 31 and 32, and regulating valves (control valves) 33 and 34. The flow meter 31 and the regulating valve 33 are disposed in the passage 103. The flow meter 32 and the regulating valve 34 are disposed in the passage 104. The flow meter 31 measures the flow rate of the heating medium that flows in the passage 103. The data measured by the flow meter 31 (measured value of the flow rate of the heating medium that flows in the passage 103) is sent to the control unit 15. The flow meter 32 measures the flow rate of the heat medium that flows in the passage 104. The data measured by the flow meter 32 (measured value of the flow rate of the heating medium that flows in the passage 104), is sent to the control unit 15. The regulating valve 33 is a valve for adjusting the flow rate of the heating medium that flows in the passage 103. The regulating valve 34 is a valve for adjusting the flow rate of the heating medium that flows in the passage 104.

The control unit 15 may determine a set value V1 of the flow rate of the heating medium that passes through the reactor 1, based on the capacity of the reactor 1. The control unit 15 may determine a set value V2 of the flow rate of the heating medium that flows in the passage 103, and a set value V3 of the flow rate of the heating medium that flows in the passage 104, based on the set value V1 and the capacity of the reactor 2. The capacity of the reactor 1, the capacity of the reactor 2, and the set values V1, V2 and V3 may be stored in a storage unit (e.g. memory) of the control unit 15. The control unit 15 may control the flow rate of the heating medium that flows in the passage 103, based on the set value V2. The control unit 15 may control the flow rate of the heating medium that flows in the passage 104, based on the set value V3. The control unit 15 is an example of the flow rate control unit. The control unit 15 may monitor the measured value of the flow rate of the heating medium that flows in the passage 103, and adjust the flow rate of the heating medium that flows in the passage 103 by controlling the opening of the regulating valve 33. The control unit 15 may monitor the measured value of the flow rate of the heating medium that flows in the passage 104, and adjust the flow rate of the heating medium that flows in the passage 104 by controlling the opening of the regulating valve 34.

The generation device 100 includes a temperature adjusting unit 35 and a thermometer 36 which are disposed in the circulation passage 102. The temperature adjusting unit 35 adjusts the temperature of the heating medium that flows in the circulation passage 102. The temperature adjusting unit 35 may be disposed in the circulation passage 102 on the upstream side of the reactor 1, and on the downstream side of a merged portion of the passage 103 and the passage 104. In this case, the temperature adjusting unit 35 adjusts the temperature of the heating medium that flows in the circulation passage 102 on the upstream side of the reactor 1, and on the downstream side of the merged portion of the passage 103 and the passage 104. The temperature adjusting unit 35 is an example of the second temperature adjusting unit. The temperature adjusting unit 35 may include at least one of a heater which heats the heating medium that flows in the circulation passage 102 and a cooler which cools the heating medium that flows in the circulation passage 102. Since the heating medium, of which temperature has been adjusted by the temperature adjusting unit 35, is delivered to the reactor 1, it becomes easier to appropriately maintain the temperature inside the reactor 1.

The thermometer 36 measure the temperature of the heating medium that flows in the circulation passage 102. The thermometer 36 may be disposed in the circulation passage 102 on the upstream side of the reactor 1, and on the downstream side of the merged portion of the passage 103 and the passage 104. The thermometer 36 may measure the temperature of the heating medium that flows in the circulation passage 102 on the upstream side of the reactor 1, and on the downstream side of the merged portion of the passage 103 and the passage 104. In the configuration example illustrated in Fig. 2, the thermometer 36 is disposed in the circulation passage 102 on the upstream side of the reactor 1, and on the downstream side of the temperature adjusting unit 35. In this case, the thermometer 36 measures the temperature of the heating medium that flows in the circulation passage 102 on the upstream side of the reactor 1, and on the downstream side of the temperature adjusting unit 35. The data measured by the thermometer 36 (measured values of the temperature of the heating medium) is sent to the control unit 15. The control unit 15 acquires the data measured by the thermometer 36.

The control unit 15 controls the temperature adjusting unit 14 based on the data measured by the thermometer 25. The temperature adjusting unit 14 adjusts the temperature of the heating medium such that the temperature of the heating medium that flows in the passage 103 on the upstream side of the reactor 2 falls within a first temperature range. The control unit 15 controls the temperature adjusting unit 35 based on the data measured by the thermometer 36. The temperature adjusting unit 35 adjusts the temperature of the heating medium such that the temperature of the heating medium that flows in the circulation passage 102 on the upstream side of the reactor 1, and on the downstream side of the merged portion of the passage 103 and the passage 104 falls within a second temperature range, which is higher than the first temperature range. This makes it easier to maintain the temperature inside the reactor 1 at a temperature higher than the temperature inside the reactor 2.

The first temperature range and the second temperature range are temperature ranges that can be set freely. The first temperature range and the second temperature range may be determined by experiment or simulation. The first temperature range and the second temperature range may be stored in the storage unit of the control unit 15. The first temperature range and the second temperature range may also be determined considering the reaction amount and the reaction speed of the reactor 1, the reaction amount and the reaction speed of the reactor 2, and the like. The first temperature range may be a temperature range that can maintain the inside of the reactor 2 at a low temperature state, for example. The second temperature range may be a temperature range that can maintain the inside of the reactor 1 at a high temperature state, for example.

According to the generation device 100 of Embodiment 2, the heating medium of which temperature has been adjusted passes through the reactor 2 first, and then passes through the reactor 1. Hence it can be prevented that the temperature of the heating medium, which passes through the reactor 2 becomes excessively high, and this makes it easier to appropriately maintain the temperature inside the reactor 2. By maintaining the inside of the reactor 2 at an appropriate temperature (e.g. low temperature), the concentration of the product gas, generated in the reactor 2, becomes higher due to chemical equilibrium, and the generation device 100 can generate high concentration product gas.

### <Embodiment 3>

Embodiment 3 will be described. In Embodiment 3, a composing element the same as Embodiments 1 and 2 is denoted with a same reference sign, and description thereof will be omitted. The generation devices 100 according to Embodiments 1 to 3 may be combined as needed.

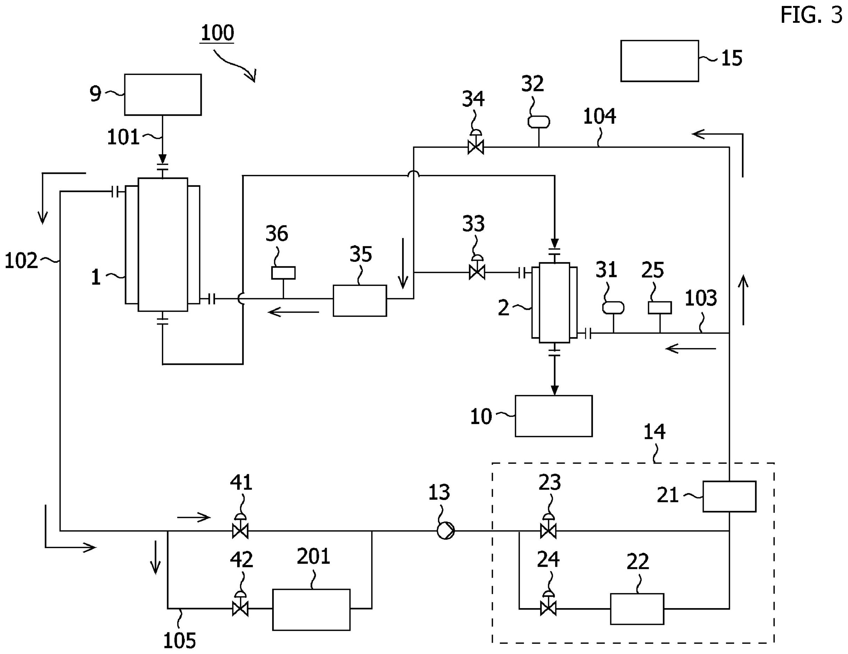

Fig. 3 is a block diagram of the generation device 100 according to Embodiment 3. Compared with the generation device 100 according to Embodiment 2, the generation device 100 according to Embodiment 3 further includes regulating valves 41 and 42 and a passage 105. The passage 105 branches from the circulation passage 102 on the downstream side of the reactor 1, and merges with the circulation passage 102 on the upstream side of the temperature adjusting unit 14. An equipment unit 201 is disposed in the passage 105. A plurality of equipment units 201 may be disposed in the passage 105. At least a part of the heating medium that flows in the circulation passage 102 on the downstream side of the reactor 1 and on the upstream side of the reactor 2 flows in the passage 105. The heating medium that flows in the passage 105 flows into the circulation passage 102 through the equipment unit 201.

The regulating valve 41 is disposed in the circulation passage 102, and is disposed between a branched portion from the circulation passage 102 and a merged portion with the circulation passage 102. The regulating valve 41 is a valve for adjusting the flow rate of the heating medium which flows in the circulation passage 102 on the downstream side of the reactor 1 and on the upstream side of the reactor 2. The regulating valve 42 is disposed in the passage 105. The regulating valve 42 is a valve for adjusting the flow rate of the heating medium which flows in the passage 105.

The control unit 15 controls the regulating valves 41 and 42. In a case where the regulating valve 41 closes and the regulating valve 42 opens, the heating medium is delivered to the temperature adjusting unit 14 through the equipment unit 201. In a case where the regulating valve 41 opens and the regulating valve 42 closes, the heating medium is delivered to the temperature adjusting unit 14 bypassing the equipment unit 201. By the opening/closing of the regulating valves 41 and 42, the heating medium that flows in the circulation passage 102 can be delivered to the temperature adjusting unit 14 through the equipment unit 201, or can be delivered to the temperature adjusting unit 14 bypassing the equipment unit 201.

The flow rate of the heating medium that flows in the circulation passage 102 may be adjusted by the regulating valve 41, and the flow rate of the heating medium that flows in the passage 105 may be adjusted by the regulating valve 42. Thereby a part of the heating medium that flows in the circulation passage 102 is delivered to the temperature adjusting unit 14 through the equipment unit 201, and a part of the heating medium that flows in the circulation passage 102 is delivered to the temperature adjusting unit 14 bypassing the equipment unit 201.

The equipment unit 201 may be a factory equipment unit, a heating equipment unit, or the like, which utilizes the heat of the heating medium that flows in the passage 105. The heating medium which passed through the reactor 1 is at high temperature, hence the heat of the heating medium which passed through the reactor 1 can be used for the equipment unit 201. The heating medium that flows in the passage 105 is cooled by the equipment unit 201, and is returned in this state to the downstream side of the temperature adjusting unit 14 in the circulation passage 102.

### <Embodiment 4>

Embodiment 4 will be described. In Embodiment 4, a composing element the same as Embodiments 1 and 2 is denoted with a same reference sign, and description thereof will be omitted. The generation devices 100 according to Embodiments 1 to 4 may be combined as needed.

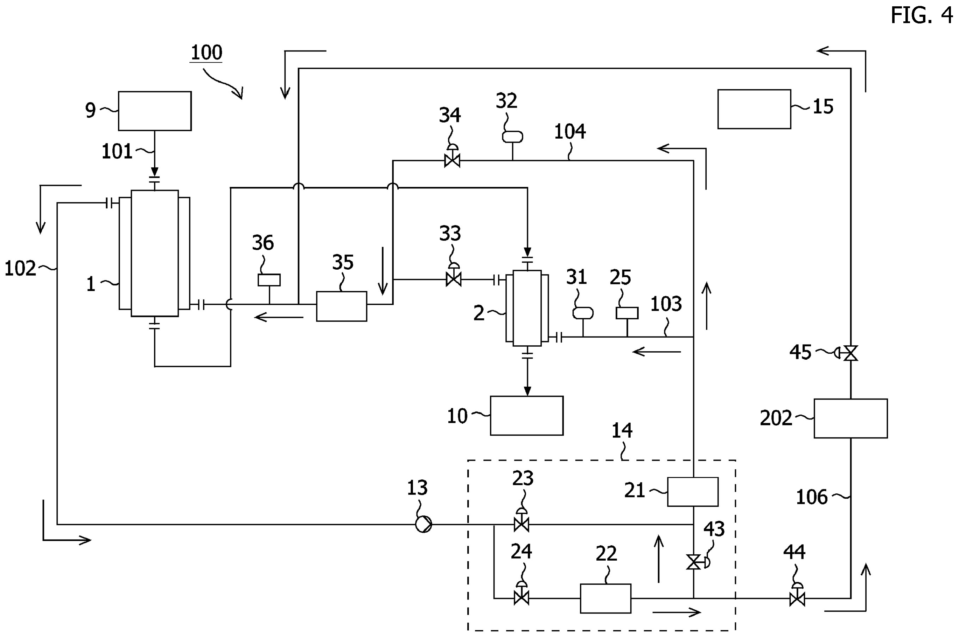

Fig. 4 is a block diagram of the generation device 100 according to Embodiment 4. Compared with the generation device 100 according to Embodiment 2, the generation device 100 according to Embodiment 4 further includes regulating valves 43, 44 and 45 and a passage 106. The passage 106 branches from the circulation passage 102 on the upstream side of the reactor 2, and merges with the circulation passage 102 on the upstream side of the reactor 1 and on the downstream side of the reactor 2. In the configuration example illustrated in Fig. 4, the passage 106 merges with the circulation passage 102 on the downstream side of the merged portion of the passage 103 and the passage 104. An equipment unit 202 is disposed in the passage 106. A plurality of equipment units 202 may be disposed in the passage 106. A part of the heating medium that flows in the circulation passage 102 on the downstream side of the reactor 1 and on the upstream side of the reactor 2 flows in the passage 106. The heating medium that flows in the passage 106 flows into the circulation passage 102 through the equipment unit 202.

The regulating valve 43 is disposed in the circulation passage 102. The regulating valves 44 and 45 are disposed in the passage 106. The regulating valve 43 is a valve for adjusting the flow rate of the heating medium which flows in the circulation passage 102 on the downstream side of the reactor 1 and on the upstream side of the reactor 2. The regulating valves 44 and 45 are valves for adjusting the flow rate of the heating medium which flows in the passage 106.

The control unit 15 controls the regulating valves 23, 24, 43, 44 and 45. In a case where the regulating valves 23, 24, 44 and 45 open and the regulating valve 43 closes, a part of the heating medium that flows in the circulation passage 102 flows into the passage 106 through the cooler 22. In a case where the regulating valve 23 closes and the regulating valves 24, 43, 44 and 45 open, a part of the heating medium that flows in the circulation passage 102 is delivered to the heater 21 through the cooler 22, and a part of the heating medium that flows in the circulation passage 102 flows into the passage 106 through the cooler 22.

The equipment unit 202 may be a factory equipment unit, a cooling equipment unit, or the like, which utilizes the heating medium that flows in the passage 106. The heating medium which passed through the cooler 22 has been cooled, hence the heating medium which passed through the cooler 22 can be used for the equipment unit 202. The heating medium that flows in the passage 106 is heated by the equipment unit 202, and is returned in this state to the circulation passage 102 on the upstream side of the reactor 1 and on the downstream side of the reactor 2. Thereby the heating medium heated by the equipment unit 202 can be delivered to the reactor 1.

### <Embodiment 5>

Embodiment 5 will be described. In Embodiment 5, a composing element the same as Embodiment 1 is denoted with a same reference sign, and description thereof will be omitted. The generation devices 100 according to Embodiments 1 and 3 to 5 may be combined as needed.

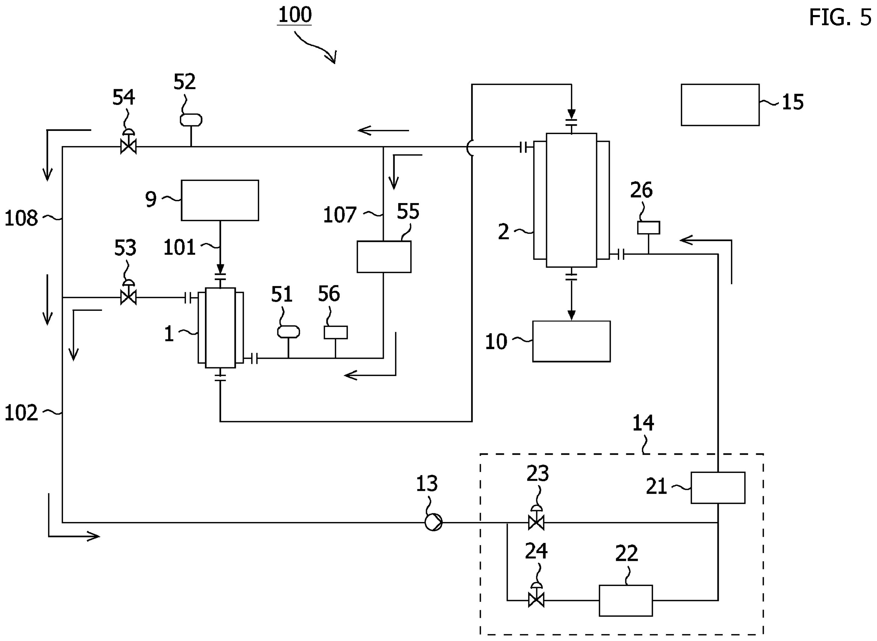

Fig. 5 is a block diagram of the generation device 100 according to Embodiment 5. The generation device 100 includes reactors 1 and 2, economizers 3 and 4, gas cooling heat exchangers 5 and 6, gas-liquid separators 7 and 8, a source gas supply unit 9, and a storage tank 10. The generation device 100 includes a drain valve 11, a chiller 12, a pump 13, a temperature adjusting unit 14, a control unit 15, a gas passage 101, and a circulation passage 102. In Fig. 5, the economizers 3 and 4, the gas cooling heat exchangers 5 and 6, the gas-liquid separators 7 and 8, the drain valve 11 and the chiller 12 are omitted.

In Embodiment 5, the capacity of the reactor 1 and the capacity of the reactor 2 are different, and the capacity of the reactor 2 is larger than the capacity of the reactor 1. The ratio of the capacity of the reactor 1 and the capacity of the reactor 2 is not especially limited. The ratio of the capacity of the reactor 1 and the capacity of the reactor 2 may be determined by experiment or simulation. The circulation passage 102 includes: a passage (first passage) 107 in which the heating medium flows through the reactor 1; and a passage (second passage) 108 which branches from the passage 107, and in which the heating medium flows bypassing the reactor 1. The passage 108 merges with the passage 107 on the downstream side of the reactor 2 in the passage 107.

In Embodiment 5, the capacity of the reactor 2 is larger than the capacity of the reactor 1, hence the amount of product gas generated by the reactor 2 increases compared with Embodiments 1 to 4, and the reactor 2 can produce a larger amount of high concentration product gas.

In Embodiment 5, the capacity of the reactor 1 is smaller than the capacity of the reactor 2. If the capacity of the reactor 1 decreases, the surface area of the reactor 1 decreases. As the surface area of the reactor 1 is smaller, the heat release amount of the reactor 1 decreases. By decreasing the surface area of the reactor 1, heat released from the reactor 1 can be suppressed, which makes it easier to appropriately maintain the temperature inside the reactor 1. Further, if the surface area of the reactor 1 is decreased, the apparatus cost (e.g. cost of material, cost of insulating material) of the reactor 1 can be reduced.

In Embodiment 5, the flow rate of the heating medium which passes through the reactor 1 and the flow rate of the heating medium which passes through the reactor 2 are set based on the difference between the capacity of the reactor 1 and the capacity of the reactor 2. In the case where the capacity of the reactor 1 is smaller than the capacity of the reactor 2, it is preferable that the flow rate of the heating medium which passes through the reactor 1 is lower than the flow rate of the heating medium which passes through the reactor 2. For example, in the case where the flow rate of the heating medium which flows into the shell of the reactor 1 is excessively high, a pressure loss is generated, and load on the pump 13 increases thereby. In Embodiment 5, the circulation passage 102 includes a passage 107 in which the heating medium flows through the reactor 1, and a passage 108 in which the heating medium flows bypassing the reactor 1. Thereby the flow rate of the heating medium which passes through the reactor 1 decreases and the pressure loss is suppressed, and load on the pump 13 is decreased. As a result, power to be supplied to the pump 13 can be reduced.

The generation device 100 includes a thermometer 26 which is disposed in the circulation passage 102. The thermometer 26 measures the temperature of the heating medium that flows in the circulation passage 102. The thermometer 26 may be disposed in the circulation passage 102 on the upstream side of the reactor 2. The thermometer 26 measures the temperature of the heating medium that flows on the upstream side of the reactor 2 in the circulation passage 102. In the configuration example illustrated in Fig. 5, the thermometer 26 is disposed in the circulation passage 102 on the upstream side of the reactor 2, and on the downstream side of the temperature adjusting unit 14. In this case, the thermometer 26 measures the temperature of the heating medium that flows in the circulation passage 102 on the upstream side of the reactor 2, and on the downstream side of the temperature adjusting unit 14. The data measured by the thermometer 26 (measured value of the temperature of the heating medium) is sent to the control unit 15. The control unit 15 acquires the data measured by the thermometer 26.

The generation device 100 includes flow meters 51 and 52, and regulating valves (control valves) 53 and 54. The flow meter 51 and the regulating valve 53 are disposed in the passage 107. The flow meter 52 and the regulating valve 54 are disposed in the passage 108. The flow meter 51 measures the flow rate of the heating medium that flows in the passage 107. The data measured by the flow meter 51 (measured value of the flow rate of the heating medium that flows in the passage 107) is sent to the control unit 15. The flow meter 52 measures the flow rate of the heating medium that flows in the passage 108. The data measured by the flow meter 52 (measured value of the flow rate of the heating medium that flows in the passage 108) is sent to the control unit 15. The regulating valve 53 is a valve for adjusting the flow rate of the heating medium that flows in the passage 107. The regulating valve 54 is a valve for adjusting the flow rate of the heating medium that flows in the passage 108.

The control unit 15 may determine a set value V4 of the flow rate of the heating medium that passes through the reactor 2, based on the capacity of the reactor 2. The control unit 15 may determine a set value V5 of the flow rate of the heating medium that flows in the passage 107, and a set value V6 of the flow rate of the heating medium that flows in the passage 108, based on the set value V4 and the capacity of the reactor 1. The capacity of the reactor 1, the capacity of the reactor 2, and the set values V4, V5 and V6, may be stored in a storage unit of the control unit 15. The control unit 15 may control the flow rate of the heating medium that flows in the passage 107, based on the set value V5. The control unit 15 may control the flow rate of the heating medium that flows in the passage 108, based on the set value V6. The control unit 15 may monitor the measured value of the flow rate of the heating medium that flows in the passage 107, and adjust the flow rate of the heating medium that flows in the passage 107 by controlling the opening of the regulating valve 53. The control unit 15 may monitor the measured value of the flow rate of the heating medium that flows in the passage 108, and adjust the flow rate of the heating medium that flows in the passage 108 by controlling the opening of the regulating valve 54.

The generation device 100 according to Embodiment 5 includes a temperature adjusting unit 55 and a thermometer 56 which are disposed in the passage 107. The temperature adjusting unit 55 adjusts the temperature of the heating medium that flows in the passage 107. The temperature adjusting unit 55 may be disposed in the passage 107 on the upstream side of the reactor 1, and on the downstream side of a branched portion of the passage 107 and the passage 108. In this case, the temperature adjusting unit 55 adjusts the temperature of the heating medium that flows in the passage 107 on the upstream side of the reactor 1, and on the downstream side of the branched portion of the passage 107 and the passage 108. The temperature adjusting unit 55 is an example of the second temperature adjusting unit. The temperature adjusting unit 55 may include at least one of a heater which heats the heating medium that flows in the passage 107, and a cooler which cools the heating medium that flows in the passage 107. Since the heating medium of which temperature has been adjusted by the temperature adjusting unit 55, is delivered to the reactor 1, it becomes easier to appropriately maintain the temperature inside the reactor 1.

The thermometer 56 measures the temperature of the heating medium that flows in the passage 107. The thermometer 56 may be disposed in the passage 107 on the upstream side of the reactor 1, and on the downstream side of the branched portion of the passage 107 and the passage 108. The thermometer 56 measures the temperature of the heating medium that flows in the passage 107 on the upstream side of the reactor 1, and on the downstream side of the branched portion of the passage 107 and the passage 108. In the configuration example illustrated in Fig. 5, the thermometer 56 is disposed in the passage 107 on the upstream side of the reactor 1, and on the downstream side of the temperature adjusting unit 55. In this case, the thermometer 56 measures the temperature of the heating medium that flows in the passage 107 on the upstream side of the reactor 1, and on the downstream side of the temperature adjusting unit 55. The data measured by the thermometer 56 (measured value of the temperature of the heating medium) is sent to the control unit 15. The control unit 15 acquires the data measured by the thermometer 56.

The control unit 15 controls the temperature adjusting unit 14 based on the data measured by the thermometer 26. The temperature adjusting unit 14 adjusts the temperature of the heating medium such that the temperature of the heating medium that flows in the circulation passage 102 on the upstream side of the reactor 2 falls within a first temperature range. The control unit 15 controls the temperature adjusting unit 55 based on the data measured by the thermometer 56. The temperature adjusting unit 55 adjusts the temperature of the heating medium such that the temperature of the heating medium that flows in the passage 107 on the upstream side of the reactor 1, and on the downstream side of the branched portion of the passage 107 and the passage 108 falls within a second temperature range, which is higher than the first temperature range. This makes it easier to maintain the temperature inside the reactor 1 at a temperature higher than the temperature inside the reactor 2.

The first temperature range and the second temperature range are temperature ranges that can be set freely. The first temperature range and the second temperature range may be determined by experiment or simulation. The first temperature range and the second temperature range may be stored in the storage unit of the control unit 15. The first temperature range and the second temperature range may also be determined considering the reaction amount and the reaction speed of the reactor 1, the reaction amount and the reaction speed of the reactor 2, and the like. The first temperature range may be a temperature range that can maintain the inside of the reactor 2 at a low temperature state, for example. The second temperature range may be a temperature range that can maintain the inside of the reactor 1 at a high temperature state, for example.

According to the generation device 100 of Embodiment 5, the heating medium, of which temperature has been adjusted, passes through the reactor 2 first, and then passes through the reactor 1. Hence it can be prevented that the temperature of the heating medium which passes through the reactor 2 becomes excessively high, and this makes it easier to appropriately maintain the temperature inside the reactor 2. By maintaining the inside of the reactor 2 at an appropriate temperature (e.g. low temperature), the concentration of the product gas generated in the reactor 2 becomes higher due to chemical equilibrium, and the generation device 100 can generates high concentration product gas.

### <Modification>

The modification of the above Embodiments 1 to 5 described will be described next. In this modification, a composing element the same as Embodiment 1 is denoted with a same reference sign, and description thereof will be omitted. The generation device 100 according to this modification can be applied to the generation devices 100 according to Embodiment 1 to 5. The generation device 100 according to this modification includes a plurality of (at least three) reactors, including the reactors 1 and 2.

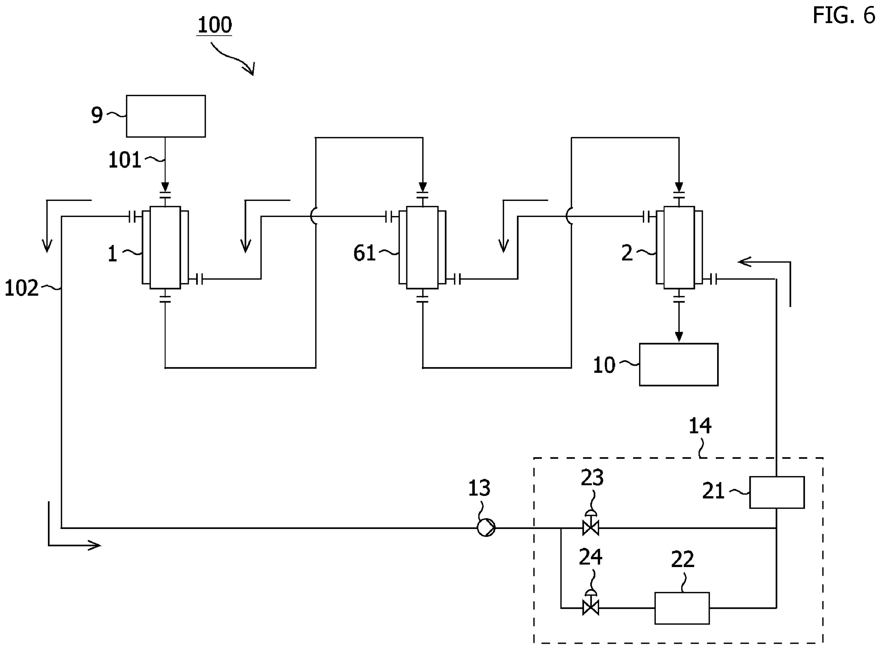

Fig. 6 is a block diagram of the generation device 100 according to this modification. The generation device 100 according to this modification includes reactors 1 and 2, a reactor 61 which is disposed between the reactor 1 and the reactor 2, economizers 3 and 4, gas cooling heat exchangers 5 and 6, gas-liquid separators 7 and 8, a source gas supply unit 9, and a storage tank 10. The generation device 100 according to this modification includes a drain valve 11, a chiller 12, a pump 13, a temperature adjusting unit 14, a control unit 15, a gas passage 101, and a circulation passage 102. In Fig. 6, the economizers 3 and 4, the gas cooling heat exchangers 5 and 6, the gas-liquid separators 7 and 8, the drain valve 11, and the chiller 12 are omitted.

In the direction in which the source gas flows in the gas passage 101, the reactor 61 is disposed behind the reactor 1, and the reactor 2 is disposed behind the reactor 61. This means that in the direction in which the source gas flows in the gas passage 101, the reactor 1 is disposed first, and the reactor 2 is disposed last. Further, in the direction in which the heating medium circulates in the circulation passage 102, the reactor 2 is disposed behind the temperature adjusting unit 14, the reactor 61 is disposed behind the reactor 2, and the reactor 1 is disposed behind the reactor 61. The generation device 100 may include another reactor disposed between the reactor 1 and the reactor 61, or include another reactor disposed between the reactor 2 and the reactor 61.

The heating medium of which temperature has been adjusted by the temperature adjusting unit 14 passes through the reactor 2 first, and then passes through the reactor 61. In this modification, the heating medium of which temperature has been adjusted passes through the reactor 2 first, and then passes through the reactor 61, hence it can be prevented that the temperature of the heating medium which passes through the reactor 2 becomes excessively high, and this makes it easier to appropriately maintain the temperature inside the reactor 2. By maintaining inside the reactor 2 at an appropriate temperature (e.g. low temperature), the concentration of the product gas generated in the reactor 2 becomes higher due to chemical equilibrium, and the generation device 100 can generate high concentration product gas.

### REFERENCE SIGNS

1, 2, 61 Reactor; 3, 4 Economizer; 5, 6 Gas cooling heat exchanger; 7, 8 Gas-liquid separator; 9 Source gas supply unit; 10 Storage tank; 11 Drain valve; 12 Chiller; 13 Pump; 14, 35, 55 Temperature adjusting unit; 15 Control unit; 21 Heater; 22 Cooler; 23, 24 Regulating valve; 25, 26, 36, 56 Thermometer; 31, 32, 51, 52 Flowmeter; 33, 34, 41, 42, 43, 44, 45, 53, 54 Regulating valve; 100 Generation device; 101 Gas passage; 102 Circulation passage; 103, 104, 105, 106, 107, 108 Passage; 201, 202 Equipment unit

## Claims

1. A generation device comprising:
a plurality of reactors that include a first reactor and a second reactor to generate product gas by catalytic reaction of source gas;
a gas passage in which the source gas flows through the plurality of reactors;
a circulation passage in which heating medium for adjusting temperature inside the plurality of reactors circulates through the plurality of reactors; and
a temperature adjusting unit which is disposed in the circulation passage and adjusts temperature of the heating medium, wherein
in a direction in which the source gas flows in the gas passage, the second reactor is disposed behind the first reactor, and
in a direction in which the heating medium circulates in the circulation passage, the second reactor is disposed behind the temperature adjusting unit, and the first reactor is disposed behind the second reactor.

2. The generation device according to claim 1, wherein
capacity of the first reactor and capacity of the second reactor are different.

3. The generation device according to claim 1, wherein
capacity of the second reactor is smaller than capacity of the first reactor.

4. The generation device according to claim 1, wherein
capacity of the second reactor is larger than capacity of the first reactor.

5. The generation device according to claim 3, wherein
the circulation passage includes a first passage in which the heating medium, of which temperature has been adjusted by the temperature adjusting unit, flows through the second reactor, and a second passage, which branches from the first passage, and in which the heating medium, of which temperature has been adjusted by the temperature adjusting unit, flows bypassing the second reactor, and
the second passage merges with the first passage on the downstream side of the second reactor.

6. The generation device according to claim 5, further comprising:
a flow rate control unit that control a flow rate of the heating medium which flows in the first passage and a flow rate of the heating medium which flows in the second passage.

7. The generation device according to claim 5, further comprising:
a second temperature adjusting unit which is disposed in the circulation passage, on the upstream side of the first reactor, and on the downstream side of a merged portion of the first passage and the second passage, and which adjusts the temperature of the heating medium.

8. The generation device according to claim 7, wherein
the temperature adjusting unit adjusts the temperature of the heating medium such that the temperature of the heating medium which flows in the first passage on the upstream side of the second reactor falls within a first temperature range, and
the second temperature adjusting unit adjusts the temperature of the heating medium such that the temperature of the heating medium which flows on the upstream side of the first reactor and on the downstream side of the merged portion falls within a second temperature range, which is higher than the first temperature range.

9. The generation device according to claim 4, wherein
the circulation passage includes a first passage in which the heating medium flows through the first reactor, and a second passage which branches from the first passage and in which the heating medium flows bypassing the first reactor, and
the second passage merges with the first passage on the downstream side of the first reactor.

10. The generation device according to claim 9, further comprising:
a flow rate control unit that controls a flow rate of the heating medium which flows in the first passage and a flow rate of the heating medium which flows in the second passage.

11. The generation device according to claim 9, further comprising:
a second temperature adjusting unit which is installed in the circulation passage, and is disposed in the first passage on the upstream side of the first reactor and on the downstream side of a branched portion of the first passage and the second passage, and which adjusts the temperature of the heating medium.

12. The generation device according to claim 11, wherein
the temperature adjusting unit adjusts the temperature of the heating medium such that the temperature of the heating medium which flows on the upstream side of the second reactor falls within a first temperature range, and
the second temperature adjusting unit adjusts the temperature of the heating medium such that the temperature of the heating medium which flows in the first passage on the upstream side of the first reactor and on the downstream side of the branched portion falls within a second temperature range, which is higher than the first temperature range.

13. The generation device according to claim 1, wherein
at least a part of the heating medium that flows in the circulation passage on the downstream side of the first reactor and on the upstream side of the second reactor is returned to the downstream side of the temperature adjusting unit in the circulation passage, in a state of being cooled by at least one equipment unit.

14. The generation device according to claim 1, wherein
the temperature adjusting unit includes a cooler that cools the heating medium, and
a part of the heating medium cooled by the cooler is returned to the circulation passage on the upstream side of the first reactor and on the downstream side of the second reactor, in a state of being heated by at least one equipment unit.

15. The generation device according to any one of claims 1 to 14, wherein
the source gas flows inside the plurality of reactors from gas inlets located on a first side of the plurality of reactors to gas outlets located on a second side of the plurality of reactors, and
the heating medium flows inside the plurality of reactors from heating medium inlets located on the second side of the plurality of reactors to heating medium outlets located on the first side of the plurality of reactors.
